# EUROPÄISCHE PATENTSCHRIFT

(11) **EP 3 010 438 B1**
(45) Veröffentlichungstag und Bekanntmachung des Hinweises auf die Patenterteilung: **09.08.2017**
(21) Anmeldenummer: 14734002.0
(22) Anmeldetag: 20.06.2014
(51) Int. Cl.: A61B 90/30, F21S 8/04, F21W 131/205

(54) **VERFAHREN FÜR DIE KALIBRIERUNG EINER SENSORVORRICHTUNG FÜR DIE REGELUNG DER AUSRICHTUNG EINER AUSLEUCHTVORRICHTUNG UND AUSLEUCHTSYSTEM**
METHOD FOR CALIBRATING A SENSOR DEVICE FOR REGULATING THE ALIGNMENT OF AN ILLUMINATION DEVICE AND ILLUMINATION SYSTEM
PROCÉDÉ D'ÉTALONNAGE D'UN SYSTÈME DE CAPTEUR SERVANT À RÉGLER L'ORIENTATION D'UN DISPOSITIF D'ÉCLAIRAGE ET SYSTÈME D'ÉCLAIRAGE

(30) Priorität: 22.06.2013 DE 102013010513
(43) Veröffentlichungstag der Anmeldung: 27.04.2016
(73) Patentinhaber: Drägerwerk AG & Co. KGaA, 23558 Lübeck (DE)
(72) Erfinder: SCHLICHTING, Stefan, Dr., 23562 Lübeck (DE); SCHLAEFER, Alexander, 23562 Lübeck (DE); HARTMANN, Florian, 23558 Lübeck (DE)
(74) Vertreter: Mildner, Volker
(86) Internationale Anmeldenummer: PCT/EP2014/001689
(87) Internationale Veröffentlichungsnummer: WO 2014/202229

(56) Entgegenhaltungen:
- EP-A1- 2 283 790
- BENNET TILLAPAUGH ET AL: "Indirect camera calibration for surgery tracking", BIOMEDICAL IMAGING: FROM NANO TO MACRO, 2009. ISBI '09. IEEE INTERNATIONAL SYMPOSIUM ON, IEEE, PISCATAWAY, NJ, USA, 28. Juni 2009 (2009-06-28), Seiten 1083-1086, XP031502236, ISBN: 978-1-4244-3931-7

## Beschreibung

Die vorliegende Erfindung betrifft ein Verfahren für die Kalibrierung einer Sensorvorrichtung für die Regelung der Ausrichtung einer Ausleuchtvorrichtung zur Veränderung der Position eines durch die Ausleuchtvorrichtung in einer Ausleuchtrichtung erzeugten Ausleuchtbereichs sowie ein entsprechendes Ausleuchtsystem mit einer solchen Ausleuchtvorrichtung, einer Sensorvorrichtung und einer entsprechenden Kontrolleinheit.

Es ist grundsätzlich bekannt, dass z. B. in einem Operationssaal automatisiert verstellbare Ausleuchtsysteme vorgesehen werden. Diese weisen eine Ausleuchtvorrichtung in Form einer Operationslampe auf. Mithilfe dieser Operationslampe als Ausleuchtvorrichtung kann in Ausleuchtrichtung ein Ausleuchtbereich erzeugt werden. Für den Einsatz einer solchen Ausleuchtvorrichtung muss der Ausleuchtbereich an einen definierten Ort verschoben werden. Dies geschieht bei bekannten Ausleuchtvorrichtungen durch eine manuelle Verstellung der Ausleuchtrichtung, um den Ausleuchtbereich mit einem gewünschten auszuleuchtenden Bereich zur Deckung zu bringen.

Nachteilhaft bei bekannten Ausleuchtvorrichtungen ist es, dass die manuelle Einflussnahme auf die Ausleuchtvorrichtung einen Griff des Bedienpersonals an die Ausleuchtvorrichtung erfordert. Wird die Ausleuchtvorrichtung z. B. in einem sterilen Raum, z. B. in einem OP-Bereich, eingesetzt, so bedeutet dieser Handgriff eine Bewegung aus dem Steril bereich heraus.

Dementsprechend ist bereits vorgeschlagen worden, dass über Gestensteuerungen eine automatisierte Verstellung des Ausleuchtbereichs erfolgen kann. Jedoch erfordert eine automatisierte Verstellung eine Aktuierung hinsichtlich der Verstellung der Ausrichtung der Ausleuchtvorrichtung. Hierfür ist z. B. ein Roboterarm vorgesehen, welcher in seinem Koordinatensystem Stellbewegungen zur Veränderung der Ausleuchtrichtung der Ausleuchtvorrichtung durchführt. Gleichzeitig muss eine Kontrolle bzw. eine Vorgabe einer Sensorvorrichtung erfolgen, um die reale Veränderung der Ausleuchtrichtung und damit eine Verschiebung des Ausleuchtbereichs zu erzielen, zu erzeugen bzw. zu überwachen. Hierfür sind bekannterweise Sensorvorrichtungen, z. B. in Form von Kamerasystemen, im Einsatz.

Nachteilhaft bei den bereits bekannten automatisierten Veränderungsverfahren für die Ausleuchtvorrichtungen ist die aufwendige Kalibrierung. So muss zwischen dem Koordinatensystem der Sensorvorrichtung einerseits und dem Koordinatensystem der Verstelleinheit für die Ausleuchtvorrichtung andererseits eine Transformationsmatrix ermittelt werden, um die beiden Koordinatensysteme miteinander in Einklang bringen zu können. Eine solche Transformationsmatrix ist zwingend notwendig, um eine entsprechende Regelstrecke zur Verfügung stellen zu können. Bei bekannten Ausleuchtvorrichtungen ist die Kalibration, also die Erzeugung einer solchen Transformationsmatrix, mit sehr hohem Aufwand besetzt.

Es ist daher Aufgabe der vorliegenden Erfindung, die voranstehend beschriebenen Nachteile zumindest teilweise zu beheben. Insbesondere ist es Aufgabe der vorliegenden Erfindung, in einfacher und kostengünstiger Weise insbesondere eine zeitsparendes, bevorzugt ein automatisierbares Kalibrierverfahren zur Verfügung zu stellen.

Voranstehende Aufgabe wird gelöst durch ein erfindungsgemäßes Verfahren mit den Merkmalen des Anspruchs 1 sowie durch ein erfindungsgemäßes Ausleuchtsystem mit den Merkmalen des Anspruchs 12. Weitere Merkmale und Details der Erfindung ergeben sich aus den Unteransprüchen, der Beschreibung und den Zeichnungen. Dabei gelten Merkmale und Details, die im Zusammenhang mit dem erfindungsgemäßen Verfahren beschrieben sind, selbstverständlich auch im Zusammenhang mit dem erfindungsgemäßen Ausleuchtsystem und jeweils umgekehrt, sodass bezüglich der Offenbarung zu den einzelnen Erfindungsaspekten stets wechselseitig Bezug genommen wird bzw. werden kann.

Ein erfindungsgemäßes Verfahren dient der Kalibrierung einer Sensorvorrichtung für die Regelung der Ausrichtung einer Ausleuchtvorrichtung. Dabei dient es der Veränderung der Position eines durch die Ausleuchtvorrichtung in einer Ausleuchtrichtung erzeugten Ausleuchtbereichs. Ein erfindungsgemäßes Verfahren weist die folgenden Schritte auf:
a) Erzeugen eines von der Ausleuchtvorrichtung in der Ausleuchtrichtung ausgehenden Lichtpunktes auf einer Kalibrierfläche,
b) Definieren eines ersten Kalibrierpunktes als Mittelpunkt einer Kugeloberfläche in der Ausleuchtrichtung,
c) Erkennen der Position des Lichtpunktes auf der Kalibrierfläche mit der Sensorvorrichtung,
d) Verändern der Ausrichtung der Ausleuchtvorrichtung entlang der Kugeloberfläche,
e) Wiederholtes Durchführen der Schritte c) und d) unter Erzeugung von wenigstes drei unterschiedlichen Positionen des Lichtpunktes auf der Kalibrierfläche,
f) Bestimmen der Abstände der unterschiedlichen Positionen des Lichtpunktes,
g) Wiederholtes Durchführen der Schritte b) bis f), bis zum Erreichen eines Abruchkriteriums,
h) Definieren des ersten Kalibrierpunktes und der erkannten Position des Lichtpunktes auf der Kalibrierfläche als Korrespondenzpunkt zur Beziehung zwischen dem Koordinatensystem der Sensorvorrichtung (110) und dem Koordinatensystem der Ausleuchtvorrichtung,
i) Definieren wenigstens eines weiteren Kalibrierpunktes als Mittelpunkt einer Kugeloberfläche in der Ausleuchtrichtung,
j) Durchführen der Schritte b) bis h) für den wenigstens einen weiteren Kalibrierpunkt.

Ein erfindungsgemäßes Verfahren dient also der Kalibrierung der Sensorvorrichtung, vorzugsweise in automatisierbarer Art. Dabei wird eine Korrespondenz zwischen der Erkennungsmöglichkeit der Sensorvorrichtung und der Beeinflussungsmöglichkeit der Ausleuchtvorrichtung festgestellt. Kern der vorliegenden Erfindung ist es, einen Lichtpunkt zur Verfügung zu stellen. Unter einem Lichtpunkt ist dabei insbesondere ein Laserlichtpunkt zu verstehen. Grundsätzlich sind jedoch auch andere Lichtpunkte im Rahmen der vorliegenden Erfindung denkbar. Von Vorteil ist es, wenn die geometrische Erstreckung des Lichtpunktes möglichst klein gehalten wird. Aufgrund des vorbestimmten Abstandes zwischen der Ausleuchtvorrichtung und der Kalibrierfläche ist dementsprechend eine Lichtquelle für die Erzeugung des Lichtpunktes von Vorteil, welche eine möglichst geringe Auffächerung bzw. eine möglichst große Fokussierung des erzeugten Lichtstrahls auf die Kalibrierfläche ermöglicht.

Ein Kalibrierpunkt ist im Sinne der vorliegenden Erfindung ein virtueller Punkt vor oder hinter der Kalibrierfläche. Er wird als realer Punkt im realen Koordinatensystem definiert. Dieser erste Kalibrierpunkt dient als Vergleichspunkt für die erkannten Positionen des Lichtpunktes auf der Kalibrierfläche. Im Laufe des erfindungsgemäßen Verfahrens wird dieser Kalibrierpunkt auf die Kalibrierfläche zu bewegt so dass sich damit der Mittelpunkt der entsprechenden Kugeloberfläche ebenfalls auf die Kalibrierfläche zubewegt.

Erfindungsgemäß werden einzelne Schritte schleifenweise wiederholt. So wird in einer ersten kleinen Schleife (Schritte c und d) eine Veränderung der Position des Lichtpunktes auf der Kalibrierfläche erzeugt. Damit ergibt sich für diese Schleife nach mehrmaligem Durchlauf durch die unterschiedlichen Punkte auf der Kalibrierfläche ein Muster bzw. eine geometrische Form. Zum Beispiel handelt es sich dabei um ein Dreieck, ein Rechteck, ein Quadrat oder eine ähnliche Form.

In einer zweiten, mittleren Schleife werden die Schritte b) bis f) wiederholt durchgeführt, so dass für jeden Durchlauf der ersten, kleinen Schleife jeweils eine geometrische Form auf der Kalibrierfläche durch die verschiedenen Punkte entsteht. Aufgrund der Tatsache, dass es sich bei dem Kalibrierpunkt um den Mittelpunkt einer Kugeloberfläche in der Ausleuchtrichtung handelt, erfolgt die Veränderung der Ausleuchtvorrichtung auf dieser Kugeloberfläche. Die geometrische Form der ersten, kleinen Schleife erlaubt durch seine Größe einen Rückschluss auf den Abstand zwischen dem Kalibrierpunkt und der Kalibrierfläche.

Je größer der Abstand zwischen dem Kalibrierpunkt und der Kalibrierfläche ausgebildet ist, umso größer ist der entsprechende Abstand zwischen den einzelnen Punkten der geometrischen Form. Die tatsächliche Ausgestaltung der geometrischen Form ist dabei im Wesentlichen unerheblich. Das Abruchkriterium für die zweite, mittlere Schleife berücksichtigt dabei insbesondere den jeweils bestimmten Abstand zwischen den Positionen des Lichtpunktes auf der Kalibrierfläche, also der Erstreckung der jeweiligen geometrischen Form. Dabei wird für die einzelnen Durchläufe der zweiten, mittleren Schleife insbesondere immer die gleiche oder im Wesentlichen die gleiche geometrische Form erzeugt. Die verbessert die Vergleichbarkeit der einzelnen Durchläufe und damit die Geschwindigkeit bei der Durchführung eines erfindungsgemäßen Verfahrens.

Wird das Abbruchkriterium erreicht, so werden der Kalibrierpunkt und die erkannte Position des Lichtpunktes als Korrespondenzpunkt der Sensorvorrichtung und der Ausleuchtvorrichtung gesetzt. Mit anderen Worten besteht nunmehr für diesen Punkt eine Korrespondenz in den unterschiedlichen Koordinatensystemen der Sensorvorrichtung und der Ausleuchtvorrichtung. Während der Lichtpunkt in seiner Position im Koordinatensystem der Sensorvorrichtung erkannt wird, besteht gleichzeitig eine entsprechende Korrespondenz zur Position der Ausleuchtvorrichtung. Diese Korrespondenz wirkt sich dahingehend aus, dass nunmehr die bestimmte Ausrichtung der Ausleuchtvorrichtung, also die Positionierung der Ausleuchtvorrichtung mit dem erreichten Abbruchkriterium, einen Lichtpunkt erzeugt, welcher mit dem definierten Kalibrierpunkt übereinstimmt bzw. unter Berücksichtigung des Abbruchkriteriums im Wesentlichen übereinstimmt. Dieser erste Korrespondenzpunkt stellt nunmehr eine Beziehung her zwischen dem Koordinatensystem der Ausleuchtvorrichtung einerseits und dem Koordinatensystem der Sensorvorrichtung andererseits. Somit kann der Korrespondenzpunkt Eingang finden in die notwendige Transformationsmatrix zwischen diesen beiden Koordinatensystemen der Sensorvorrichtung und der Ausleuchtvorrichtung.

Um sicherzustellen, dass die Transformationsmatrix auch bei anderen Koordinatensystemen ausreichende Eingabewerte beinhaltet, wird wenigstens ein weiterer Kalibrierpunkt gesetzt, für welchen die beschriebenen Schritte b) bis h) nochmals durchgeführt werden. Bevorzugt ist es, dass für wenigstens drei Kalibrierpunkte die Schritte b) bis h) insgesamt durchgeführt werden. Damit kann im dreidimensionalen Koordinatensystem auch eine dreidimensionale Transformationsmatrix erzeugt werden, um die erfindungsgemäß zur Verfügung stellbare Kalibrierung zwischen Sensorvorrichtung und Ausleuchtvorrichtung erzeugen zu können.

Unter einer Sensorvorrichtung ist im Sinne der vorliegenden Erfindung insbesondere eine Kameravorrichtung und/oder eine Kameraeinheit zu verstehen. Die Ausleuchtvorrichtung ist insbesondere eine Operationsleuchtvorrichtung, die zur Ausleuchtung eines Operationsausleuchtbereichs Einsatz finden soll. Dementsprechend ist der Ausleuchtbereich insbesondere ein Operationsausleuchtbereich.

Die Kalibrierfläche kann durch einen separaten Kalibrierkörper zur Verfügung gestellt werden. Jedoch ist es auch möglich, dass die Kalibrierfläche bereits z. B. durch einen vorhandenen Tisch, insbesondere einen Operationstisch, vorhanden ist. Die Kalibrierfläche ist vorzugsweise angepasst an eine ausreichende Reflexion des Lichtes, welches den Lichtpunkt ausbildet. Damit wird sichergestellt, dass der Lichtpunkt auch in der gewünschten und für das Verfahren notwendigen Weise von der Sensorvorrichtung wahrgenommen werden kann. Der Lichtpunkt ist das Ergebnis der Projektion eines Lichtstrahls mit vorzugsweise nur geringem Auffächerungsgrad. Insbesondere wird Laserlicht, vorzugsweise im Wesentlichen kohärentes Licht verwendet.

Weiter ist es möglich, dass für die Erkennung der Position eine Aufspaltung in das grundsätzliche Erkennen des Lichtpunktes, z. B. durch eine Farbkamera, und das Positionieren des erkannten Punktes, z. B. durch eine Tiefenkamera, erfolgen kann, insbesondere wenn die Transformation zwischen Kamera und Tiefenkamera bekannt ist, d.h. Kamera und Tiefenkamera sind zueinander kalibriert.

Ein Abbruchkriterium ist im Wesentlichen frei bestimmbar. So kann allein durch eine vorbestimmte Anzahl an Schleifenwiederholungen ein Abbruchkriterium zur Verfügung gestellt werden. Dabei handelt es sich also um eine maximale Iterationszahl der entsprechenden Schleife. Auch das Erreichen einer minimalen Abweichung und dementsprechend einer Korrespondenzgenauigkeit zwischen dem erkannten Lichtpunkt und dem zugehörigen Kalibrierpunkt ist ein Abbruchkriterium im Sinne der vorliegenden Erfindung.

Die Punktkorrespondenzen werden, wie in beschriebener Weise, für die Erzeugung der Transformationsmatrix zwischen Sensorvorrichtung und Ausleuchtvorrichtung verwendet. Um in einem dreidimensionalen Koordinatensystem eine entsprechend ausreichend bestimmte Transformationsmatrix zur Verfügung zu stellen, sind vorzugsweise insgesamt drei Kalibrierpunkte mit den entsprechenden Verfahrensschritten belegt worden.

Eine Veränderung der Ausrichtung der Ausleuchtvorrichtung erfolgt durch eine Bewegung der Ausleuchtvorrichtung auf der Kugeloberfläche, für welche der zugehörige Kalibrierpunkt den Mittelpunkt bildet. Mit anderen Worten erfolgt eine winklige Veränderung der Ausleuchtrichtung, so dass alle Ausleuchtrichtungen im Verlauf der ersten, kleinen Schleife immer durch den jeweiligen aktuellen Kalibrierpunkt verlaufen.

Ein erfindungsgemäßes Verfahren wird vorzugsweise nach der Montage des entsprechenden Ausleuchtsystems durchgeführt. Vorzugsweise wird es jedoch häufiger, z. B. mindestens einmal täglich, durchgeführt, um eine möglicherweise entstehende Verstellung der Sensorvorrichtung und/oder der Ausleuchtvorrichtung durch Rekalibration ausgleichen zu können. Ein erfindungsgemäßes Verfahren wird vorzugsweise automatisiert durchgeführt, sodass insbesondere Verfahrensdurchläufe für alle Iterationszahlen von weniger als ca. 1 Minute erzielbar werden.

Ein erfindungsgemäßes Verfahren lässt sich dahingehend weiterbilden, dass der Lichtpunkt in Form eines Laserlichtpunktes erzeugt wird. Insbesondere wird ein Laser mit kohärentem Licht verwendet. Dies erlaubt es, auch größere Abstände zwischen der Ausleuchtvorrichtung und der Kalibrierfläche zuzulassen. Gleichzeitig wird eine möglichst punktförmige Ausbildung des Lichtpunktes gewährleistet, um auch in definierter Weise möglichst genau den Abstand zwischen der Position des Lichtpunktes und dem Kalibrierpunkt definieren zu können. Insbesondere folgt eine Anpassung der Oberfläche der Kalibrierfläche an die Wellenlänge des Lasers und/oder die Art der Sensorvorrichtung. Dabei handelt es sich insbesondere um eine Anpassung hinsichtlich der Reflexionseigenschaften der Kalibrierfläche.

Vorteilhaft ist es ebenfalls, wenn bei einem erfindungsgemäßen Verfahren der Schritt des Erkennens der Position des Lichtpunktes auf der Kalibrierfläche auf zwei Sensoreinheiten der Sensorvorrichtung aufgeteilt ist. Dabei erkennt eine erste Sensoreinheit den Lichtpunkt auf der Kalibrierfläche und eine zweite Sensoreinheit bestimmt den Ort des erkannten Lichtpunktes auf der Kalibrierfläche. Die erste Sensoreinheit kann dabei ein optisches Kamerasystem z. B. eine Farbkamera und die zweite Sensoreinheit z. B. eine Tiefenkamera sein. Auch bereits bestehende Ausleuchtsysteme können auf diese Weise nachgerüstet werden. So kann es vorteilhaft sein, wenn bereits bei einer vorhandenen Tiefenkamera durch die Nachrüstung einer Farbkamera die Vorrichtungsgegebenheiten für die Durchführung eines erfindungsgemäßen Verfahrens nachgerüstet werden. Darüber hinaus können die jeweiligen Sensoreinheiten an die spezifische Aufgabe angepasst sein. Insbesondere ist es möglich, die zweite Sensoreinheit auch für die Durchführung eines Regelverfahrens bei einer Gestensteuerung während der Verwendung der Ausleuchtvorrichtung einzusetzen. Somit kann ein Teil der grundsätzlich notwendigen Regelungseinheiten, nämlich die zweite Sensoreinheit, auch für ein erfindungsgemäßes Kalibrierverfahren zur Verfügung gestellt werden. Dies reduziert die Komplexität der Vorrichtung und die damit einhergehenden Kosten.

Ebenfalls von Vorteil kann es sein, wenn bei einem erfindungsgemäßen Verfahren als Abbruchkriterium wenigstens eines der folgenden eingesetzt wird:
- Vorgabe einer maximalen Wiederholungszahl
- Vorgabe eines maximalen Abstandes zwischen den Lichtpunkten bezüglich eines Kalibrierpunkts

Es wird also vorzugsweise als Abbruchkriterium z. B. ein Genauigkeitswert in Form eines vordefinierten ε vorgegeben. Damit wird ein maximaler Abstand zwischen den erkannten Positionen des Lichtpunktes mit Bezug zu dem jeweiligen Kalibrierpunkt vordefiniert, welcher im Wesentlichen als Positionierung des Kalibrierpunkts in der Kalibrierfläche und dementsprechend als Korrespondenzpunkt gesetzt wird. Selbstverständlich können die beiden voranstehend beschriebenen Abbruchkriterien auch miteinander kombiniert werden, sodass eine maximale Wiederholungszahl eine Unendlichkeitsschleife des Verfahrens verhindert. Gleichzeitig muss jedoch nicht die maximale Wiederholungszahl erreicht werden, wenn bereits vorher eine Reduktion des Abstandes und dem vordefinierten maximalen Abstand erfolgen konnte. Somit kann einerseits der Genauigkeitsgrad der Kalibrierung vorgegeben werden, während allgemein eine Zeitobergrenze für die Durchführung des Verfahrens definierbar wird. Die Vorgabe kann dabei allgemein oder auch spezifisch für jede Kalibrierung durchgeführt werden. Insbesondere sind solche Vorgabewerte zumindest grundsätzlich allgemein gespeichert, sodass die Durchführung des Verfahrens automatisiert erfolgen kann.

Vorteilhaft ist es darüber hinaus, wenn bei einem erfindungsgemäßen Verfahren die Veränderung der Ausrichtung der Ausleuchtvorrichtung nach einem vorgegebenen Muster erfolgt. Mit anderen Worten sind die Veränderungsbewegungen der Ausleuchtvorrichtung durch dieses Muster vorgegeben. Es erfolgt also eine zielgerichtete Veränderung der Ausrichtung durch definiertes Verändern des Winkels der Ausleuchtrichtung auf der Kugeloberfläche mit einem Mittelpunkt im jeweiligen Kalibrierpunkt. Das Muster ist insbesondere abhängig von der Zahl der Wiederholungen, sodass für jeden Iterationsschritt eine Veränderung des Musters vorgenommen werden kann. Die Veränderung des Musters zielt dabei insbesondere auf eine Erhöhung der Genauigkeit bzw. eine Reduktion des Abstandes zwischen den erzeugten Lichtpunkten und dem Kalibrierpunkt hin. Damit wird durch die Vorgabe eines Musters eine noch leichtere Automatisierung eines erfindungsgemäßen Kalibrierverfahrens möglich. Unter einem Muster ist insbesondere eine geometrische Form zu verstehen, welche durch die verschiedenen Positionen auf der Kalibrierfläche des Lichtpunktes erzeugt werden. Dabei kann es sich zum Beispiel um ein Dreieck, ein Rechteck oder ähnliche zweidimensionale Formen handeln. Bei der Veränderung der Ausrichtung der Ausleuchtvorrichtung wird dabei insbesondere eine Veränderung der zugehörigen Winkelkoordinaten bzw. der zugehörigen Polarkoordinaten durchgeführt.

Ebenfalls von Vorteil ist es, wenn bei einem Verfahren gemäß dem voranstehenden Absatz es sich bei dem Muster um ein Suchfeld mit gleichen oder im Wesentlichen gleichen Abständen der einzelnen Feldpunkte zueinander handelt. Das Suchfeld entspricht dabei der bereits mehrfach beschriebenen geometrischen Form. Diese geometrische Form wird bestimmt durch eine gedachte Linienverbindung zwischen den Positionen des Lichtpunktes auf der Kalibrierfläche bezogen auf jeweils einen Durchlauf der ersten, kleinen Schleife. Das Suchfeld wird dabei insbesondere durch entsprechende Polarkoordinaten und/oder Winkelkoordinaten auf der beschriebenen Kugeloberfläche definiert. Während der Variation der Ausleuchtrichtung innerhalb der ersten, kleinen Schleife erfolgt eine Bewegung der Ausleuchtvorrichtung auf der durch den jeweiligen Kalibrierpunkt als Mittelpunkt bestimmten Kugeloberfläche.

Bei einem erfindungsgemäßen Verfahren gemäß dem voranstehenden Absatz ist es weiter möglich, wenn nach Abfahren aller Feldpunkte des Suchfeldes die Reihenfolge bei der Erkennung der Position des Lichtpunktes auf der Kalibrierfläche berücksichtigt wird, um die relative Position des ersten Kalibrierpunktes zur Kalibrierfläche zu bestimmen. Auf diese Weise kann die notwendige Richtung der Verschiebung des Kalibrierpunktes bestimmt werden. Je nach Richtung des Erzeugens der geometrischen Position durch die einzelnen Lichtpunkte kann auf diese Weise bestimmt werden, ob der Kalibrierpunkt sich vor oder hinter der Kalibrierfläche befindet. Damit wird sichergestellt, dass sich der Kalibrierpunkt nach dem Überschreiten der Kalibrierfläche sozusagen wieder in die entgegengesetzte Richtung der Kalibrierfläche annähert. Damit wird im Verlauf der zweiten, mittleren Schleife sozusagen ein Einpendeln auf die Kalibrierfläche möglich.

Ein weiterer Vorteil ist erzielbar, wenn bei einem erfindungsgemäßen Verfahren bei der Wiederholung der Schritte b) bis f) der erste Kalibrierpunkt als Mittelpunkt einer Kugeloberfläche jeweils auf die Kalibrierfläche zu bewegt wird, insbesondere um einen vordefinierten Wert oder in Abhängigkeit einer vordefinierten Funktion.

Damit wird sozusagen die Schrittweite des Kalibrierpunktes auf die Kalibrierfläche zu vorgegeben. Insbesondere kann bei einer notwendigen Richtungsumkehr, wie sie im voranstehenden Absatz beschrieben worden ist, eine Reduktion der Schrittweite, zum Beispiel um einen vordefinierten Wert, insbesondere um 50%, erfolgt. Damit erfolgt somit ein Einpendeln auf die Kalibrierfläche mit immer weiter reduzierter Schrittweite.

Weiter kann es vor Vorteil sein, wenn bei einem erfindungsgemäßen Verfahren die Schritte a) bis i) insgesamt für wenigstens drei Kalibrierpunkte durchgeführt werden, welche alle in einer Ebene liegen, in welcher sich die Kalibrierfläche erstreckt. Dementsprechend kann in einem dreidimensionalen System auch eine Anzahl von drei Korrespondenzpunkten erzeugt werden, die wiederum eine ausreichende Datenbasis für die Erzeugung einer.Transformationsmatrix zwischen Sensorvorrichtung und Ausleuchtvorrichtung bieten. Selbstverständlich können jedoch auch mehr als drei Kalibrierpunkte in einem erfindungsgemäßen Verfahren Einsatz finden. Die drei Kalibrierpunkte definieren eindeutig die Kalibrierfläche. Sämtliche weiteren Kalibrierpunkte liegen ebenfalls in dieser Kalibrierfläche. Sie erhöhen die Genauigkeit eines erfindungsgemäßen Verfahrens weiter, da insbesondere eine Plausibilitätsprüfung bei der Erzeugung der Transformationsmatrix zwischen Sensorvorrichtung und Ausleuchtvorrichtung berücksichtigt werden kann.

Vorteilhaft ist es ebenfalls, wenn bei einem erfindungsgemäßen Verfahren alle definierten Korrespondenzpunkte als Eingangswerte für eine nachfolgende Regelung der Ausrichtung der Ausleuchtvorrichtung gespeichert und/oder an eine Kontrolleinheit zur Durchführung der Regelung dieser Ausrichtung übermittelt werden. Mit anderen Worten erfolgt eine Übergabe bzw. eine direkte Berechnung der bereits beschriebenen Transformationsmatrix. Diese Transformationsmatrix erlaubt eine Transformation zwischen dem Koordinatensystem der Sensorvorrichtung und dem Koordinatensystem der Ausleuchtvorrichtung. Sie beruht auf einer Korrespondenz im real vorhandenen dreidimensionalen Koordinatensystem der Kalibrierfläche. Mit anderen Worten erzeugt die Kalibration direkt oder indirekt die Transformationsmatrix. Dabei kann ein Teil des erfindungsgemäßen Verfahrens die Erstellung der Transformationsmatrix sein.

Jedoch ist es grundsätzlich ausreichend, wenn die definierten Korrespondenzpunkte einer nachfolgenden Kontrolleinheit zur Verfügung gestellt werden, welche wiederum in der Lage ist, die notwendige Transformationsmatrix zu erzeugen.

Vorteilhaft ist es weiter, wenn bei einem erfindungsgemäßen Verfahren der für den ersten Kalibrierpunkt gefundene Korrespondenzpunkt für die Durchführung der Schritte für den wenigstens einen weiteren Kalibrierpunkt verwendet wird. Das bedeutet, dass die nachfolgenden Kalibrierpunkte schneller zu einem Korrespondenzpunkt führen, da bereits eine erste Grundtendenz des Zusammenhangs zwischen Ausleuchtvorrichtung und Sensorvorrichtung besteht. Mit anderen Worten können für die weiteren Kalibrierpunkte die bereits gefundenen Ergebnisse, insbesondere die bereits gefundenen Korrespondenzpunkte zur Verbesserung und zur Beschleunigung des Verfahrens Verwendung finden. Damit kann für die nachfolgenden Iterationen für weitere Kalibrierpunkte eine Reduktion der Iterationszahl erreicht werden und damit die Durchführungsdauer und die maximale Iterationszahl für ein erfindungsgemäßes Verfahren reduziert werden.

Vorteilhaft ist es darüber hinaus, wenn bei einem erfindungsgemäßen Verfahren vor Erzeugen des Lichtpunktes ein Kalibrierkörper zur Verfügung gestellt wird, welcher die Kalibrierfläche aufweist. Dies ist insbesondere dann der Fall, wenn z. B. in einem Operationsaal kein Operationstisch vorhanden ist. Auch kann dies von Vorteil sein, wenn das entsprechende Objekt, z. B. ein Operationstisch, schlechte Reflexionswerte aufweist. Der Kalibrierkörper ist z. B. aus Metall gefertigt, um insbesondere bei Laserlicht für einen Laserlichtpunkt eine möglichst vorteilhafte Reflexion mit sich zu bringen.

Ebenfalls Gegenstand der vorliegenden Erfindung ist ein Ausleuchtsystem mit einer Ausleuchtvorrichtung, einer Sensorvorrichtung und einer Kontrolleinheit für die Regelung der Ausrichtung der Ausleuchtvorrichtung zur Veränderung der Position des durch die Ausleuchtvorrichtung in einer Ausleuchtrichtung erzeugten Ausleuchtbereichs, wobei die Sensorvorrichtung und die Kontrolleinheit für die Ausleuchtung eines erfindungsgemäßen Verfahrens ausgebildet sind.

Dementsprechend bringt ein erfindungsgemäßes Ausleuchtsystem die gleichen Vorteile mit sich, wie sie ausführlich mit Bezug auf ein erfindungsgemäßes Verfahren erläutert worden sind. Vorzugsweise weist ein erfindungsgemäßes Ausleuchtsystem eine Lichtquelle auf, um den beschriebenen Lichtpunkt zu erzeugen. Dabei handelt es sich insbesondere um eine Laserlichtquelle.

Weitere Vorteile, Merkmale und Einzelheiten der Erfindung ergeben sich aus der Beschreibung, in der unter Bezugnahme auf die Zeichnungen Ausführungsbeispiele der Erfindung im Einzelnen beschriebenen sind. Dabei können die in den Ansprüchen und der Beschreibung erwähnten Merkmale jeweils einzeln für sich oder in beliebiger Kombination erfindungswesentlich sein. Es zeigen schematisch:
- Figur 1: eine Ausführungsform eines erfindungsgemäßen Ausleuchtsystems,
- Figur 2: eine Korrelation zwischen der Ausleuchtvorrichtung und der Kalibrierfläche,
- Figur 3: eine Korrelation zwischen einem Muster und der Kalibrierfläche,
- Figur 4: ein erstes Muster,
- Figur 5: ein zweites Muster
- Figur 6: eine schematische Darstellung des Einpendelns auf die Kalibrierfläche und
- Figur 7: die schematische Darstellung der einzelnen Verfahrensschritte.

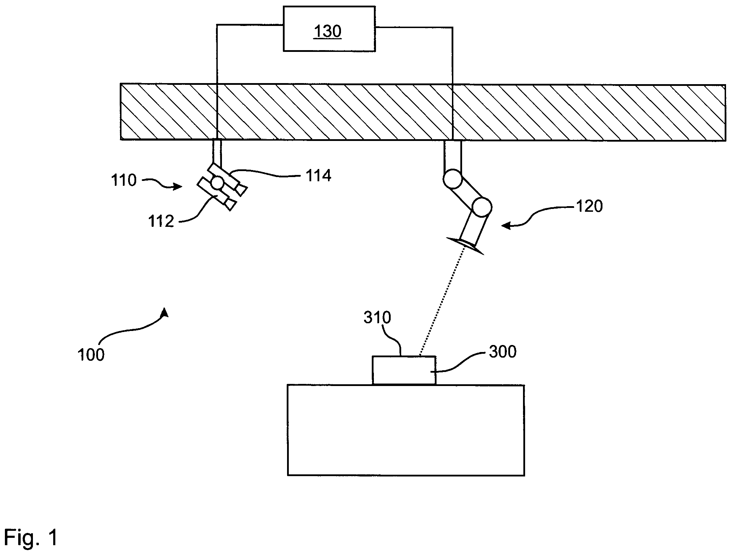

In der Figur 1 ist eine Ausführungsform eines erfindungsgemäßen Ausleuchtsystems 100 dargestellt. An der Decke eines Operationssaales ist eine Ausleuchtvorrichtung 120 bewegbar an einem Roboterarm befestigt. Darüber hinaus ist eine Sensorvorrichtung 110 mit zwei Sensoreinheiten 112 und 114 vorgesehen. Die erste Sensoreinheit 112 ist dabei als Farbkamera und die zweite Sensoreinheit 114 vorzugsweise als Tiefenkamera ausgebildet.

Weiter ist zu erkennen, dass ausgehend von der Ausleuchtvorrichtung 120 (gepunkteter Lichtstrahl) ein Lichtpunkt auf einer Kalibrierfläche 310 eines Kalibrierkörpers 300 erzeugt wird. Darüber hinaus ist eine signalkommunizierende Verbindung zwischen Ausleuchtvorrichtung 120 und einer Kontrolleinheit 130 vorgesehen. Diese Kontrolleinheit 130 steht ebenfalls in signalkommunizierender Verbindung mit der Sensorvorrichtung 110. Um nun die Sensorvorrichtung 110 mit der Ausleuchtvorrichtung 120 zu kalibrieren, erfolgt ein Kalibrationsverfahren gemäß der vorliegenden Erfindung.

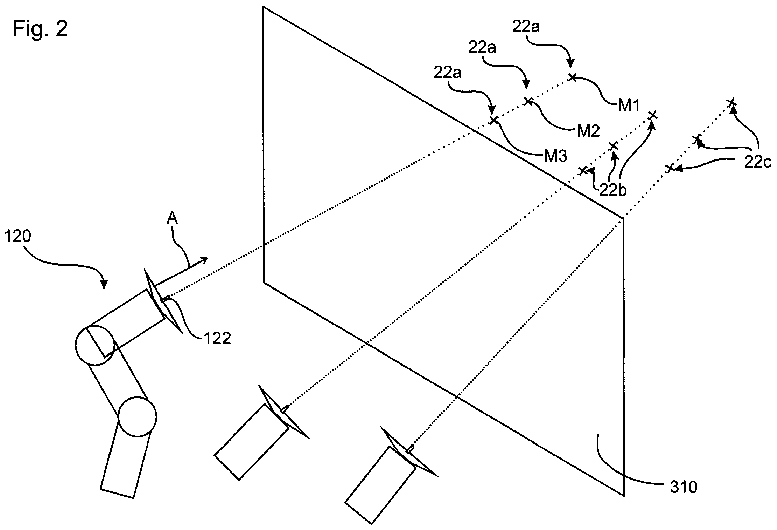

In Figur 2 ist eine perspektivische Ansicht auf die Kalibrierfläche 310 gezeigt. Hier sind die Durchläufe der einzelnen Schleifen gut zu erkennen. So wird für die erste, kleine Schleife ein ersten Kalibrierpunkt 22a definiert. Über den Verlauf der zweiten, mittleren Schleife erfolgt ein Annähern des ersten Kalibrierpunktes 22a an die Kalibrierfläche 310. Mit anderen Worten verschiebt sich über die Durchläufe der zweiten, mittleren Schleife der jeweilige Mittelpunkt M1, M2 und M3 auf die Kalibrierfläche zu. Diese Schritte der zweiten, mittleren Schleife werden bis zum Erreichen eines Abbruchkriteriums, zum Beispiel bis zum Erreichen eines minimalen Abstandes zwischen dem jeweiligen Kalibrierpunkt 22a, 22b und 22c durchgeführt.

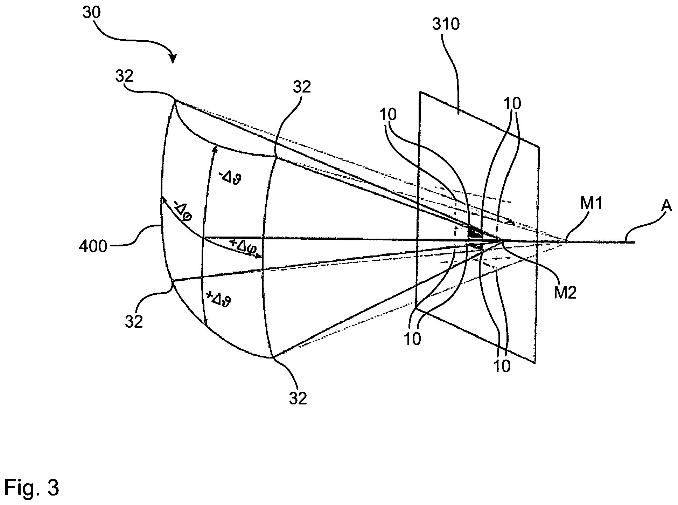

In Figur 3 wird dargestellt, wie die Veränderung der Ausleuchtrichtung innerhalb der ersten, kleinen Schleife erfolgt. Die Verschiebung verläuft dabei auf einer Kugeloberfläche, deren Mittelpunkt M1, M2, M3 der jeweilige Kalibrierpunkt 22a, 22b, 22c ist. Der Kalibrierpunkt 22a, 22b, 22c wird also im Verlauf der ersten, kleinen Schleife auf die Kalibrierfläche 310 zu bewegt. Die Erkennung des Abstandes erfolgt indirekt, nämlich über die Bestimmung der Positionen des Lichtpunktes 10 auf der Kalibrierfläche 310. Dabei wird einem Suchfeld 30 in Form eines Musters gefolgt, welches definierte Feldpunkte 32 aufweist. Hier wird als geometrische Form ein Rechteck, insbesondere ein Quadrat durch die Positionen der Lichtpunkte 10 erzeugt.

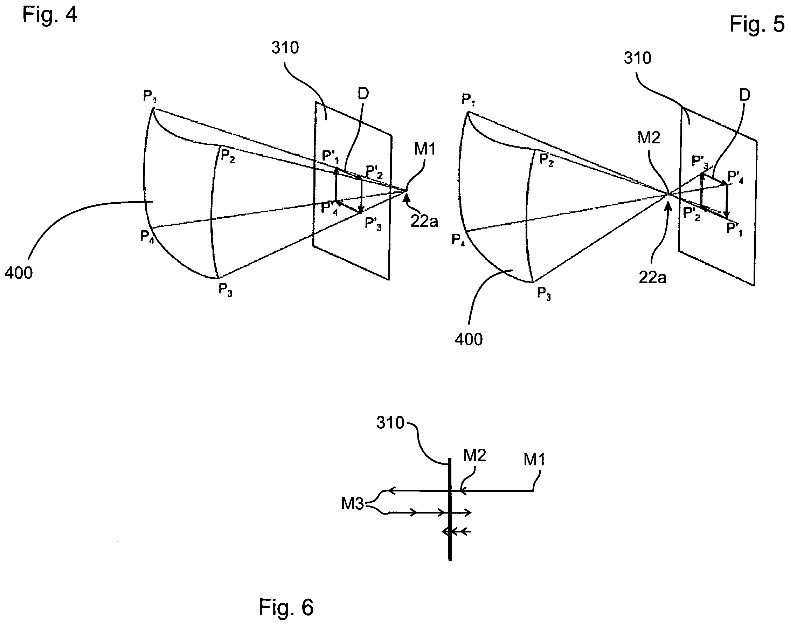

Die Figuren 4 und 5 zeigen, dass durch die Richtung, in welcher die einzelnen Positionen der Lichtpunkte 10 während der ersten, kleinen Schleife wahrgenommen werden können, einen Rückschluss auf die Richtung geben, ihn welcher der Kalibrierpunkt 22a und damit der Mittelpunkt M1 von der Kalibrierfläche 310 entfernt ist.

Die Figur 6 zeigt gut ein Einpendeln der Kalibrierpunkte 22a, 22b, 22c und damit der Mittelpunkte M1, M2, M3 auf die Kalibrierfläche 310. Ausgehend von einem ersten Mittelpunkt M1 erfolgt eine Verschiebung mit vordefinierter Schrittweite bis zum Überschreiten der Kalibrierfläche von M2 zu M3. Anschließend dreht sich die Annährungsrichtung um, so dass ein schrittweises Einpendeln der Mittelpunkte M1, M2 und M3 und damit der Kalibrierpunkte 22a, 22b, 22c erfolgen kann.

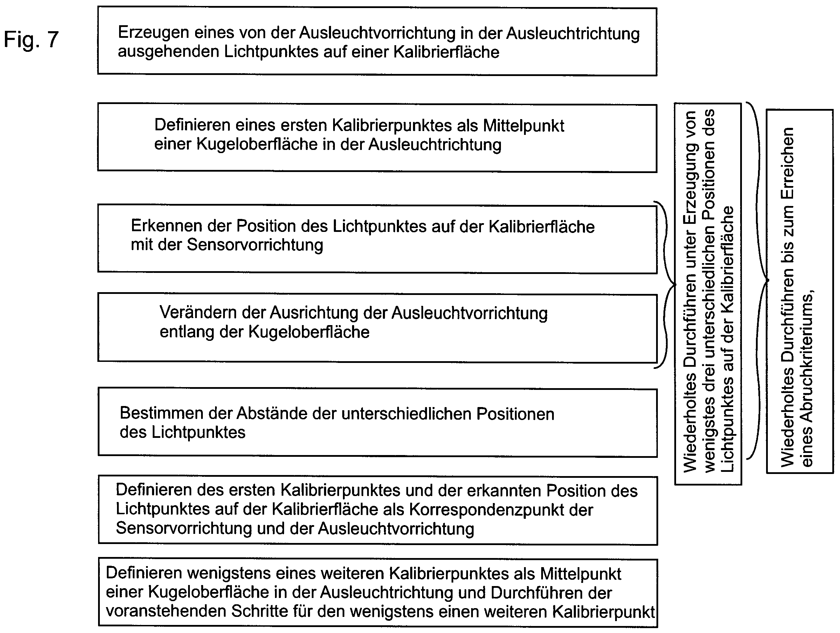

Wie z. B. der Figur 7 zu entnehmen ist, wird eine Vielzahl von zwei Schleifen durchgeführt. Die erste, kleine Schleife und die zweite, mittlere Schleife sind dabei die Durchführung von Iterationen für einen Kalibrierpunkt bis zum Erzeugen eines ersten Korrespondenzpunktes. Die große Schleife ist dabei die Durchführung dieser beiden Schleifen für insgesamt wenigstens zwei, vorzugsweise wenigstens drei Kalibrierpunkte. Dementsprechend werden insbesondere drei Korrespondenzpunkte gefunden, welche nachfolgend für die Kontrolleinheit 130 und/oder in der Kontrolleinheit 130 als Korrespondenzmatrix zur Verfügung gestellt werden können. Diese Transformationsmatrix als Korrespondenzmatrix dient der Übersetzung der erkannten Punkte durch die Sensorvorrichtung 110 in das entsprechende Koordinatensystem zur Steuerung der Ausleuchtvorrichtung 120.

Die voranstehende Erläuterung der Ausführungsformen beschreibt die vorliegende Erfindung ausschließlich im Rahmen von Beispielen. Selbstverständlich können einzelne Merkmale der Ausführungsformen, sofern technisch sinnvoll, frei miteinander kombiniert werden, ohne den Rahmen der vorliegenden Erfindung zu verlassen.

### BEZUGSZEICHENLISTE

- 10: Lichtpunkt
- 22a: erster Kalibrierpunkt
- 22b: zweiter Kalibrierpunkt
- 22c: dritter Kalibrierpunkt
- 30: Suchfeld
- 32: Feldpunkt

- 100: Ausleuchtsystem
- 110: Sensorvorrichtung
- 112: Sensoreinheit
- 114: Sensoreinheit
- 120: Ausleuchtvorrichtung
- 122: Lichtquelle des Lichtpunkts 130 Kontrolleinheit

- 200: Ausleuchtbereich

- 300: Kalibrierkörper
- 310: Kalibrierfläche

- 400: Kugeloberfläche

- A: Ausleuchtrichtung
- D: Abstand zwischen den Lichtpunkten zu einem Kalibrierpunkt
- Pn: Ausgangspunkt eines Lichtpunktes auf der Kugeloberfläche
- P'n: Lichtpunkt auf der Kalibrierfläche

- M1: Mittelpunkt
- M2: Mittelpunkt
- M3: Mittelpunkt

## Patentansprüche

1. Verfahren für die Kalibrierung einer Sensorvorrichtung (110) für die Regelung der Ausrichtung einer Ausleuchtvorrichtung (120) zur Veränderung der Position eines durch die Ausleuchtvorrichtung (120) in einer Ausleuchtrichtung (A) erzeugten Ausleuchtbereichs (200), aufweisend die folgenden Schritte:
a) Erzeugen eines von der Ausleuchtvorrichtung (120) in der Ausleuchtrichtung (A) ausgehenden Lichtpunktes (10) auf einer Kalibrierfläche (310),
b) Definieren eines ersten Kalibrierpunktes (22a) als Mittelpunkt (M1, M2, M3) einer Kugeloberfläche (400) in der Ausleuchtrichtung (120) für die Ausleuchtvorrichtung (120),
c) Erkennen der Position des Lichtpunktes (10) auf der Kalibrierfläche (310) mit der Sensorvorrichtung (110),
d) Verändern der Ausrichtung der Ausleuchtvorrichtung (120) entlang der Kugeloberfläche (400),
e) Wiederholtes Durchführen der Schritte c) und d) unter Erzeugung von wenigstes drei unterschiedlichen Positionen des Lichtpunktes (10) auf der Kalibrierfläche (310),
f) Bestimmen der Abstände (D) der unterschiedlichen Positionen des Lichtpunktes (10),
g) Wiederholtes Durchführen der Schritte b) bis f), bis zum Erreichen eines Abruchkriteriums,
h) Definieren des ersten Kalibrierpunktes (22a) und der erkannten Position des Lichtpunktes (10) auf der Kalibrierfläche (310) als Korrespondenzpunkt zur Beziehung zwischen dem Koordinatensystem der Sensorvorrichtung (110) und dem Koordinatensystem der Ausleuchtvorrichtung (120),
i) Definieren wenigstens eines weiteren Kalibrierpunktes (22b, 22c) als Mittelpunkt (M1, M2, M3) einer Kugeloberfläche (400) in der Ausleuchtrichtung (A),
j) Durchführen der Schritte b) bis h) für den wenigstens einen weiteren Kalibrierpunkt (22b, 22c).

2. Verfahren nach Anspruch 1, **dadurch gekennzeichnet, dass** der Lichtpunkt (10) in Form eines Laserlichtpunktes erzeugt wird.

3. Verfahren nach einem der vorangegangenen Ansprüche, **dadurch gekennzeichnet, dass** der Schritt des Erkennens der Position des Lichtpunktes (10) auf der Kalibrierfläche (310) auf zwei Sensoreinheiten (112, 114) der Sensorvorrichtung (110) aufgeteilt ist, wobei eine erste Sensoreinheit (112) den Lichtpunkt (10) auf der Kalibrierfläche (310) erkennt und eine zweite Sensoreinheit (114) den Ort des erkannten Lichtpunktes (10) auf der Kalibrierfläche (310) bestimmt.

4. Verfahren nach einem der vorangegangenen Ansprüche, **dadurch gekennzeichnet, dass** als Abbruchkriterium wenigstens eines der folgenden eingesetzt wird:
- Vorgabe einer maximalen Wiederholungszahl
- Vorgabe eines maximalen Abstandes (D) zwischen den Lichtpunkten bezüglich eines Kalibrierpunkts (22a, 22b, 22c)

5. Verfahren nach einem der vorangegangenen Ansprüche, **dadurch gekennzeichnet, dass** die Veränderung der Ausrichtung der Ausleuchtvorrichtung (120) nach einem vorgegebenen Muster erfolgt.

6. Verfahren nach Anspruch 5, **dadurch gekennzeichnet, dass** es sich bei dem Muster um ein Suchfeld (30) mit gleichen oder im Wesentlichen gleichen Abständen (G) der einzelnen Feldpunkte (32) zueinander handelt.

7. Verfahren nach Anspruch 6, **dadurch gekennzeichnet, dass** nach Abfahren aller Feldpunkte (32) des Suchfeldes (30) die Reihenfolge bei der Erkennung der Position des Lichtpunktes (10) auf der Kalibrierfläche (310) berücksichtigt wird, um die relative Position des ersten Kalibrierpunktes (22a, 22b, 22c) zur Kalibrierfläche (310) zu bestimmen.

8. Verfahren nach einem der vorangegangenen Ansprüche, **dadurch gekennzeichnet, dass** bei der Wiederholung der Schritte b) bis f) der erste Kalibrierpunkt (22a) als Mittelpunkt (M1, M2, M3) einer Kugeloberfläche (400) jeweils auf die Kalibrierfläche (310) zu bewegt wird, insbesondere um einen vordefinierten Wert oder in Abhängigkeit einer vordefinierten Funktion.

9. Verfahren nach einem der vorangegangenen Ansprüche, **dadurch gekennzeichnet, dass** die Schritte a) bis i) insgesamt für wenigstens drei Kalibrierpunkte (22a, 22b, 22c) durchgeführt werden.

10. Verfahren nach einem der vorangegangenen Ansprüche, **dadurch gekennzeichnet, dass** alle definierten Korrespondenzpunkte als Eingangswerte für eine nachfolgenden Regelung der Ausrichtung der Ausleuchtvorrichtung (120) gespeichert und/oder an eine Kontrolleinheit (130) zur Durchführung der Regelung dieser Ausrichtung übermittelt werden.

11. Verfahren nach einem der vorangegangenen Ansprüche, **dadurch gekennzeichnet, dass** der für den ersten Kalibrierpunkt (22a) gefundene Korrespondenzunkt für die Durchführung der Schritte für den wenigstens einen weiteren Kalibrierpunkt (22b, 22c) verwendet wird.

12. Verfahren nach einem der vorangegangenen Ansprüche, **dadurch gekennzeichnet, dass** vor Erzeugen des Lichtpunkts (10) ein Kalibrierkörper (300) zur Verfügung gestellt wird, welcher die Kalibrierfläche (310) aufweist.

13. Ausleuchtsystem (100) mit einer Ausleuchtvorrichtung (120), Sensorvorrichtung (110) und einer Kontrolleinheit (130) für die Regelung der Ausrichtung der Ausleuchtvorrichtung (120) zur Veränderung der Position des durch die Ausleuchtvorrichtung (120) in einer Ausleuchtrichtung (A) erzeugten Ausleuchtbereichs (200), wobei die Sensorvorrichtung (110) und die Kontrolleinheit (130) für die Ausführung eines Verfahrens mit den Merkmalen eines Ansprüche 1 bis 12 ausgebildet sind.

## Claims

1. A method for calibrating a sensor device (110) for regulating the alignment of an illumination device (120) for modifying the position of an illumination region (200) generated by the illumination device (120) in an illumination direction (A), having the following steps:
a) generating a light spot (10), emanating from the illumination device (120) in the illumination direction (A), on a calibration surface (310),
b) defining a first calibration point (22a) as a central point (M1, M2, M3) of a sphere surface (400) in the illumination direction (A) for the illumination device (120),
c) identifying the position of the light spot (10) on the calibration surface (310) with the sensor device (110),
d) modifying the alignment of the illumination device (120) along the sphere surface (400),
e) repeatedly performing steps c) and d) with the generation of at least three different positions of the light spot (10) on the calibration surface (310),
f) determining the distances (D) of the different positions of the light spot (10),
g) repeatedly performing steps b) to f) until a stop criterion is reached,
h) defining the first calibration point (22a) and the identified position of the light spot (10) on the calibration surface (310) as a correspondence point for the relationship between the coordinate system of the sensor device (110) and the coordinate system of the illumination device (120),
i) defining at least one further calibration point (22b, 22c) as a central point (M1, M2, M3) of a sphere surface (400) in the illumination direction (A),
j) performing steps b) to h) for the at least one further calibration point (22b, 22c).

2. A method according to claim 1, **characterised in that** the light spot (10) is generated in the form of a laser light spot.

3. A method according to one of the preceding claims,
**characterised in that** the step of identifying the position of the light spot (10) on the calibration surface (310) is split between two sensor units (112, 114) of the sensor device (110), wherein a first sensor unit (112) identifies the light spot (10) on the calibration surface (310), and a second sensor unit (114) determines the location of the identified light spot (10) on the calibration surface (310).

4. A method according to one of the preceding claims,
**characterised in that** at least one of the following is used as the stop criterion:
- specification of a maximum number of repetitions
- specification of a maximum distance (D) between the light spots with regard to a calibration point (22a, 22b, 22c).

5. A method according to one of the preceding claims,
**characterised in that** the modification of the alignment of the illumination device (120) is effected according to a predetermined pattern.

6. A method according to claim 5, **characterised in that** the pattern is a search field (30) with the same or substantially the same distances (G) of the individual field points (32) to each other.

7. A method according to claim 6, **characterised in that** after covering all the field points (32) of the search field (30) the sequence in the identification of the position of the light spot (10) on the calibration surface (310) is taken into consideration in order to determine the relative position of the first calibration point (22a, 22b, 22c) with respect to the calibration surface (310).

8. A method according to one of the preceding claims,
**characterised in that** when steps b) to f) are repeated the first calibration point (22a) is moved as a central point (M1, M2, M3) of a sphere surface (400) in each case towards the calibration surface (310), in particular by a predefined value or in a manner dependent on a predefined function.

9. A method according to one of the preceding claims,
**characterised in that** the steps a) to i) are performed in total for at least three calibration points (22a, 22b, 22c).

10. A method according to one of the preceding claims,
**characterised in that** all the defined correspondence points are stored as input values for subsequent regulation of the alignment of the illumination device (120) and/or are transmitted to a control unit (130) for performing the regulation of this alignment.

11. A method according to one of the preceding claims,
**characterised in that** the correspondence point found for the first calibration point (22a) is used for performing the steps for the at least one further calibration point (22b, 22c).

12. A method according to one of the preceding claims,
**characterised in that** prior to generating the light spot (10) a calibration body (300) is made available that has the calibration surface (310).

13. An illumination system (100) having an illumination device (120), a sensor device (110) and a control unit (130) for regulating the alignment of the illumination device (120) for modifying the position of the illumination region (200) generated by the illumination device (120) in an illumination direction (A), wherein the sensor device (110) and the control unit (130) are configured to carry out a method having the features of one of claims 1 to 12.

## Revendications

1. Procédé d'étalonnage d'un dispositif de détection (110) servant à réguler l'orientation d'un dispositif d'éclairage (120) afin de modifier la position d'une zone d'éclairage (200) produite par le dispositif d'éclairage (120) dans une direction d'éclairage (A), comportant les étapes suivantes :
a) production sur une surface d'étalonnage (310) d'un point lumineux (10) émis par le dispositif d'éclairage (120) dans la direction d'éclairage (A),
b) définition d'un premier point d'étalonnage (22a) comme centre (M1, M2, M3) d'une surface sphérique (400) dans la direction d'éclairage (120) pour le dispositif d'éclairage (120),
c) détection de la position du point lumineux (10) sur la surface d'étalonnage (310) au moyen du dispositif de détection (110),
d) modification de l'orientation du dispositif d'éclairage (120) le long de la surface sphérique (400),
e) répétition des étapes c) et d) en produisant au moins trois positions différentes du point lumineux (10) sur la surface d'étalonnage (310),
f) détermination des distances (D) des différentes positions du point lumineux (10),
g) répétition des étapes b) à f) jusqu'à atteindre un critère d'interruption,
h) définition du premier point d'étalonnage (22a) et de la position détectée du point lumineux (10) sur la surface d'étalonnage (310) comme point de correspondance pour la mise en relation du système de coordonnées du dispositif de détection (110) avec le système de coordonnées du dispositif d'éclairage (120),
i) définition d'au moins un autre point d'étalonnage (22b, 22c) comme centre (M1, M2, M3) d'une surface sphérique (400) dans la direction d'éclairage (A),
j) exécution des étapes b) à h) pour ledit au moins un autre point d'étalonnage (22b, 22c).

2. Procédé selon la revendication 1, **caractérisé en ce que** le point lumineux (10) est produit sous la forme d'un point lumineux laser.

3. Procédé selon l'une des revendications précédentes, **caractérisé en ce que** l'étape de détection de la position du point lumineux (10) sur la surface d'étalonnage (310) est répartie sur deux unités de détection (112, 114) du dispositif de détection (110), une première unité de détection (112) détectant le point lumineux (10) sur la surface d'étalonnage (310) et une deuxième unité de détection (114) déterminant l'emplacement du point lumineux (10) détecté sur la surface d'étalonnage (310).

4. Procédé selon l'une des revendications précédentes, **caractérisé en ce qu'**au moins un des critères suivants est utilisé comme critère d'interruption :
- consigne d'un nombre maximal de répétitions,
- consigne d'une distance (D) maximale entre les points lumineux par rapport à un point d'étalonnage (22a, 22b, 22c).

5. Procédé selon l'une des revendications précédentes, **caractérisé en ce que** la modification de l'orientation du dispositif d'éclairage (120) a lieu selon un motif prédéfini.

6. Procédé selon la revendication 5, **caractérisé en ce que** le motif est un champ de recherche (30) avec des distances (G) égales ou sensiblement égales des points de champ (32) individuels entre eux.

7. Procédé selon la revendication 6, **caractérisé en ce qu'**après avoir parcouru tous les points de champ (32) du champ de recherche (30), l'ordre de détection de la position du point lumineux (10) sur la surface d'étalonnage (310) est pris en compte pour déterminer la position relative du premier point d'étalonnage (22a, 22b, 22c) par rapport à la surface d'étalonnage (310).

8. Procédé selon l'une des revendications précédentes, **caractérisé en ce que** lors de la répétition des étapes b) à f), le premier point d'étalonnage (22a) défini comme centre (M1, M2, M3) d'une surface sphérique (400) est chaque fois déplacé vers la surface d'étalonnage (310), en particulier d'une valeur prédéfinie ou en fonction d'une fonction prédéfinie.

9. Procédé selon l'une des revendications précédentes, **caractérisé en ce que** les étapes a) à i) sont exécutées au total pour au moins trois points d'étalonnage (22a, 22b, 22c).

10. Procédé selon l'une des revendications précédentes, **caractérisé en ce que** tous les points de correspondance définis sont mémorisés en tant que valeurs d'entrée pour une régulation suivante de l'orientation du dispositif d'éclairage (120) et/ou transmis à une unité de contrôle (130) destinée à effectuer la régulation de cette orientation.

11. Procédé selon l'une des revendications précédentes, **caractérisé en ce que** le point de correspondance trouvé pour le premier point d'étalonnage (22a) est utilisé pour exécuter les étapes pour ledit au moins un autre point d'étalonnage (22b, 22c).

12. Procédé selon l'une des revendications précédentes, **caractérisé en ce qu'**avant la production du point lumineux (10), un corps d'étalonnage (300) qui présente la surface d'étalonnage (310) est mis à disposition.

13. Système d'éclairage (100) avec un dispositif d'éclairage (120), un dispositif de détection (110) et une unité de contrôle (130) servant à réguler l'orientation du dispositif d'éclairage (120) afin de modifier la position de la zone d'éclairage (200) produite par le dispositif d'éclairage (120) dans une direction d'éclairage (A), le dispositif de détection (110) et l'unité de contrôle (130) étant conçus pour la mise en oeuvre un procédé selon l'une des revendications 1 à 12.
